Europäisches Patentamt

European Patent Office

Office européen des brevets

(19)

(11) Publication number: **0 128 189 B1**

(12) **EUROPEAN PATENT SPECIFICATION**
**published in accordance with Art.**
**158(3) EPC**

(45) Date of publication of patent specification: **10.07.91**    (51) Int. Cl.⁵: **G01N 15/06**

(21) Application number: **84900149.0**

(22) Date of filing: **15.12.83**

(86) International application number:
**PCT/SE83/00458**

(87) International publication number:
**WO 84/02396 (21.06.84 84/15)**

(54) **METHOD AND APPARATUS FOR MEASURING THE CONTENT OF SUSPENDED SUBSTANCES IN A FLOWING MEDIUM.**

(30) Priority: **15.12.82 SE 8207180**

(43) Date of publication of application:
**19.12.84 Bulletin 84/51**

(45) Publication of the grant of the patent:
**10.07.91 Bulletin 91/28**

(84) Designated Contracting States:
**AT DE FR GB SE**

(56) References cited:
**SE-A-81 007 304**
**SE-B- 370 578**
**US-A- 3 714 444**
**US-A- 4 193 692**

(73) Proprietor: **BTG Inc.**
**2364 Park Central Boulevard**
**DECATUR, Georgia 30035-3987(US)**

(72) Inventor: **LUNDBERG, Krister**
**Trädgardsgatan 21**
**S-661 00 Säffle(SE)**
Inventor: **TIDSTAM, Göran**
**Älvstigen 5**
**S-661 00 Säffle(SE)**
Inventor: **POPE, Daniel, F.**
**625 Mount Fury Circle**
**Issaquah, WA 98027(US)**

(74) Representative: **Bjelkstam, Peter**
**Kransell & Wennborg AB Sandhamnsgatan**
**42**
**S-115 28 Stockholm(SE)**

## Description

The present invention relates to a method for measuring the content of suspended substances in a flowing medium by illuminating the medium with light from a light source, the light being detected in two detectors via detector fields, a first detector field included in a first of said detectors and arranged for measuring multiply reflected light, said light having been reflected from the substances suspended in the flowing medium, the output signals of said detectors being influenced by disturbing factors as the intensity of the light source and dirt on the glass of an insertion probe, the method providing the ratio obtained from dividing the output signals from said two detectors as a measure of the concentration of suspended substances in the flowing medium, said measure not being dependent on said disturbing factors. Additonally the invention relates to an apparatus for carrying out such a method.

A number of different methods have been developed for measuring the concentration of fibres in fibre suspensions. Methods based on mechanical apparatus have been primarily utilized. The shear force principle particularly has come into a great deal of use during the last 50 years. In shear force measurement, the network strength of the fibre suspension is measured first with the aid of a blade, a rotating sensor or the like. The relationship between the concentration and the network strength is then worked out by a special calibration method. During later years, however, optical methods have begun to be used for measuring the concentration in fibre suspensions. There are several reasons for such a development having taken place. In the first place, the mechanical meters of the shear force or viscosity type normally require a great deal of maintenance. In the second place, the accuracy, particularly with stationary measurement, is relatively poor with mechanical apparatus. In the third place, great technical development has taken place in the optical and electronic fields, particularly in the latter, which has facilitated and cheapened the manufacture of meters based on optical principles. In the fourth place, an accuracy may be obtained with electro-optical techniques which is at least as good as, and in some cases even better than, that obtained with corresponding mechanical apparatus.

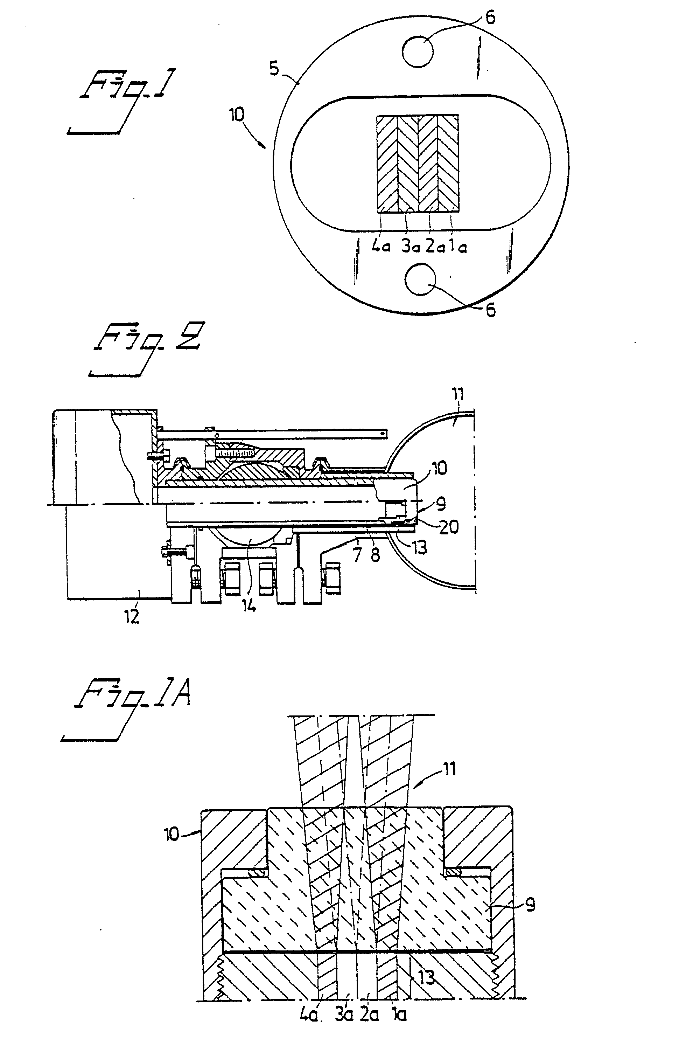

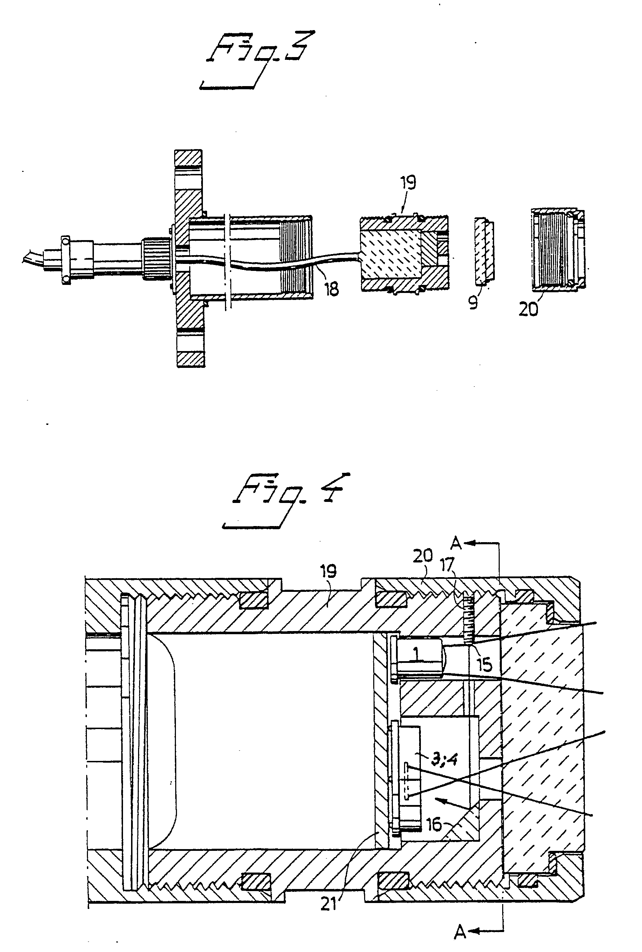

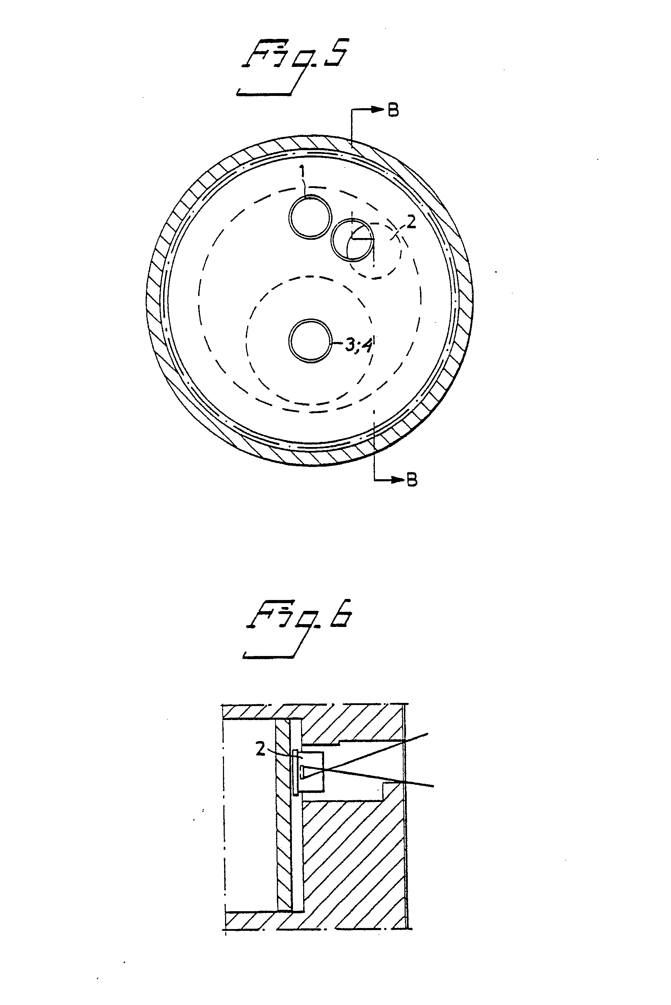

The failing in the use of electro-optical methods has, however, been the difficulty of measuring at higher concentrations. One has therefore normally been limited to measurements in the interval under 10 g/litre (1%). This is due to the fact that the primary choice has been to use transmission measurement, i.e. the light transmitted through the fibre suspension has been detected. Since the intensity of the light passing through the suspension declines according to an exponential function, the signal it provides will be close to the signal noise threshold for even relatively low concentrations. Meters based on transmitted, polarized light also suffer from the same failing. From the prior art references, i.e. US-A-3,714,444, from which the preambles of present claims 1 and 5 have been derived, a suspended solids analyzer is previously known in which multiply reflected light is measured, which is evident from the curves in Fig. 3 in said specification. Here, two detectors are used both mesuring the same type of reflection but at different distances from a light source. This analyzer comprises a plurality of photocells, each of said photocells being located at different distances from said light source and positioned to sense only light scattered backwardly from the suspended solids. From the measurement a signal is achieved which becomes non-linear for higher suspended solids concentrations and which eventually passes a curve peak and diminishes rapidly with increase in concentration. Another prior art reference is US-A-4,193,692 which refers to a method and an apparatus for the optical measurement of the concentration of a particulate in a fluid. This specification does not rely on and does not teach using and measuring directly reflected light. Here it is measured light that has been transmitted through and not reflected by the medium. The magnitude of transmitted light decreases exponentially with increasing concentration which can be seen from Fig. 3B in said specification. Here one uses clearly entirely different signals than the fact is according to the present invention. A further prior art reference is AU-A-66 809/81, which discloses an apparatus for and a method of measuring paper pulp brightness/colour and consistency. According to this specification one signal is used for the consistency measurement and the other one for brightness measurement contrary to the present invention in which both signals, directly and multiply reflected light, all are for the consistency measurement.

With the aid of the present invention as defined in claims 1 and 5, respectively, the disadvantages burdening concentration transducers known up to now have been circumvented by measuring the reflected light. In such measurements, good accuracy at concentrations well above the 1% level may be obtained, and with the present invention concentrations of 6% may be measured without deteriorated accuracy. On some types of pulp fibre good linearity continues to 10% concentration or more.

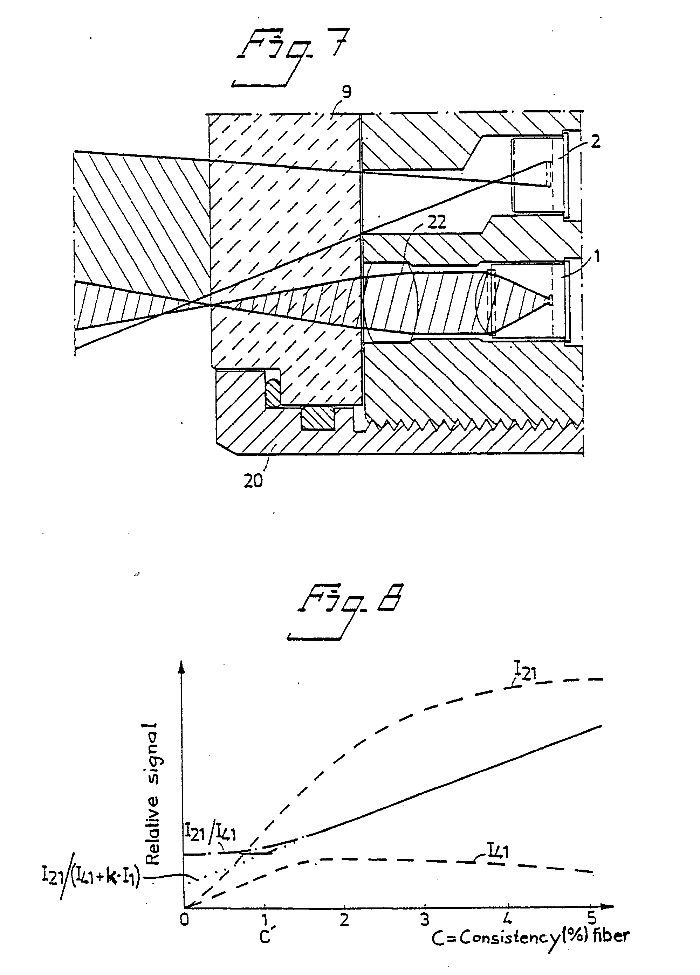

The invention will now be described below in detail with reference to the appended drawings, on which Fig. 1 is a section through a measuring head or insertion probe in a measuring apparatus according to an embodiment of the invention, Fig. 1A is a side view section of the probe showing the beam of illumination from the light source and to the detectors, Fig. 2 is a side view, partly in section, of two designs of measuring apparatus according to the invention, Fig. 3 is a side view in section, and to an enlarged scale, of an insertion probe included in another embodiment of the invention, Fig. 4 is a longitudinal section to an enlarged scale through the outer end of the insertion probe, Fig. 5 is a cross section along the line A-A through the outer end of the probe illustrated in Fig. 4, Fig. 6 is a partial section along the line B-B in the probe end illustrated in Fig. 5, Fig. 7 is a partial view of a longitudinal section through another embodiment of the probe end and Fig. 8 illustrates the signals $I_{21}$, $I_{41}$, $I_{21}/I_{41}$ and $I_{21}/(I_{41} + K \cdot I_1)$, as a function of the concentration of the flowing medium.

In the embodiment illustrated in Fig. 1, a measuring head or insertion probe 10 includes four fields, field 1a assigned to a light source 1, field 2a assigned to a detector 2 for direct reflection and fields 3a, 4a, assigned to detectors 3, 4 for multiple reflection. These fields comprise one end 13 of optical fibre conductors. The end 13 includes a window 9, behind which there is a flange 5 attached to the end portion with the aid of unillustrated attachment screws in attachment holes 6, the flange 5 being intended to centre and fix the optical fibre conductors. The apparatus functions in the following manner. Light is taken through an optical fibre conductor and out through the field 1a towards the fibre suspension flowing in a pulp line 11 (see Fig. 2). The reflected light is detected in three different detectors 2-4 via fields 2a-4a, which are at different distances from the field 1a emitting the light. The detector field 2a, which is nearest to the field 1a of the light source, primarily accepts directly reflected light for measurement by the detector 2. The other two detector fields 3a, 4a accept light for measurement by the detectors 3, 4 which is reflected a number of times in the fibre suspension and has undergone so-called multiple reflection. The beam of illumination from field 1a into stock line 11 through the window 9 is detailed in Fig. 1A, along with the angles of view of detectors 2, 3, 4 through the fields 2a, 3a, 4a. The angles of these light beams are restricted by placement of the light source and detectors with respect to the ends of the optical fibre conductors inside a casing 12.

As is shown, the angle of view detector 2 always includes some part of the direct illumination of the flowing medium regardless of the depth of penetration of this illumination, which decreases with increasing concentrations; detector 3 is barely able to receive a direct reflection except at low concentrations. As is shown by shaded lines, the angle of view for detector 4 includes practically no direct illumination of the medium at technical concentrations appropriate for this probe.

Providing that its field is sufficiently close, the detector 2, which has its field 2a closest to the light source field 1a, will respond more or less linearly to the concentration C up to a maximum proportional to the reflectance of the medium. The detector 3 will also respond linearly to C, up to a maximum, which also is proportional to the reflectance, and then will begin to decrease linearly with C. About the maximum the detector response will be approximately constant over a wide interval in concentration. The detector 4 will behave similarly to detector 3, but will reach a maximum at a lower concentration and decrease linearly with increasing C more rapidly than detector 3.

If the detectors 2, 3, 4 respond linearly to light then they will output signals proportional to $I_1$ and varying with concentration C as described above and illustrated in Fig. 8. By performing the division specified, for example $I_{21}/I_{41}$, variations in $I_1$ have no effect on the result. Also, attenuation in light caused by color in the medium or dirt on the window 9 will affect both detector outputs nearly proportionally, so again there will be no effect on the result.

As previously mentioned, the light is taken out via fibre optics towards the pulp fibres and reaches the pulp via the window 9 in the probe 10, as seen in Fig. 2. Analogously, this light is led back to an electronic transducer including detectors 2, 3, 4 situated in the casing 12. The probe 10, accommodating the fibre optics via a probe tube 8 and terminating at the common end 13, extends from the casing 12 to the pulp line 11 inside a protective tube 7. To enable cleaning the probe 10 while it is in operation, there is a sluice valve 14 in the protective tube 7, this valve being in an open position when the probe 10 is inserted, but in conjunction with the probe 10 being removed from the tube 7 the valve 14 closes and seals against the fibre suspension. In Fig. 1-2 where optical fibres are used, the respective beam path is separated by an intermediate wall in the form of a blind baffle, e.g. of metal.

The different parts forming the insertion probe 10 are apparent from the embodiment having no optical fibres, illustrated in Fig. 3-7. An electrical cable 18 (Fig. 3) terminates in an optical unit (Fig. 4), which includes the light source 1 and the detectors 2-4. Outside these there is the window 9 fastened against the unit 19 with the aid of a fastening sleeve 20.

From Fig. 4 will be apparent the way in which the window 9 is fixed with the aid of the fastening sleeve 20 to the optical unit 19, in which the light source 1 and the detector 4 are retained with the aid of a sealing fastening plate 21. A reflecting means 15 in the form of the tip of a screw 17 going through the wall of the probe 10 comes a small distance into the light flow from the light source 1. The screw tip 15 thus reflects a portion of the light coming from the light source 1 via a mirror 16 directly into the detector 4, giving rise to a signal which is added to the signal $I_{41}$ from the detector 4 according to the relationship $I_{21}/(I_{41} + k \cdot I_1)$, where the constant k is obtained optically. Instead of the reflecting means 15, an extra detector can be arranged in front of the light source 1 in its path of light, whereby the constant k in this latter case is obtained electronically. By this arrangement there is obtained the linearity for the concentration values C of the flowing medium, these values being mainly below 1%, as will be seen from the graphical depiction in Fig. 8.

From Fig. 5, which is a section through the end of the probe 10 along the line A-A in Fig. 4, will be seen the position of the light source 1 in relation to the detector 2 for the directly reflected light.

Fig. 6 is a section along the line B-B in Fig. 5 and illustrates how the light opening to the detector 2 for the directly reflected light is laterally displaced in relation to the detector 2 itself.

In the embodiment illustrated in Fig. 7, a lens system 22 has been arranged in the beam path of the light source 1 between the window 9 and said source 1. The focal length of the lens system 22 is selected such that the light going through it is focused at a point outside the window, the measuring apparatus thus being insensitive to possible dirtying of the window 9, since there is no reflection able to directly reach detector 2 from the small area of directly illuminated window surface.

Fig. 8 illustrates the different relative signals as a function of the concentration C of suspended substances in the flowing medium. It will be seen from the graphs that the relationship $I_{21}/I_{41}$ is substantially linear for fibre concentration above a critical concentration C' and that the relationship $I_{21}/(I_{41} + k \cdot I_1)$ provides a graph which is substantially linear for fibre concentrations both under and above the critical concentration C'.

Advantages obtained by adjustment of all the specified optical parameters include the extended linearity as compared with sensors using direct reflection alone, minimization of the effect of the medium color, surface dirt on window 9, and variations in light intensity $I_1$.

Electronic means for powering the light source and taking the outputs from the detectors and combining them to obtain the necessary ratios and outputting scaled signals to display or control devices should be apparent to those versed in the art.

The present embodiments use, but are not restricted to, silicon photodiodes of large area for detectors 2, 3, 4 and a gallium arsenide light emitting diode of wavelength 940 nm operating in a pulsed manner for light source 1.

**Claims**

1. A method of measuring the content of suspended substances in a flowing medium by illuminating the medium with light from a light source (1), the light being detected in two detectors (2,3;4) via detector fields (2a,3a;4a), a first detector field (4a) included in a first (4) of said detectors (2,4) and arranged for measuring multiply reflected light, said light having been reflected from the substances suspended in the flowing medium, the output signals $I_{21}, I_{41}$ of said detectors (2,3;4) being influenced by disturbing factors as the intensity of the light source and dirt on the glass of an insertion probe (10), the method providing the ratio obtained from dividing the output signals $I_{21}, I_{41}$ from said two detectors (2,4) as a measure of the concentration (C) of suspended substances in the flowing medium, said measure not being dependent on said disturbing factors, **characterized in** that a second detector field (2a) included in the second (2) of the said detectors (2,4) measures said directly reflected light, said second detector field (2a) being arranged in a predetermined position, close to the light source (1) so that directly reflected light is received and that a substantially linear relationship is obtained within the actual measuring range between the output signal $I_{21}$ from the second detector (2) and the concentration (C) of the suspended substances in the flowing medium,
in that said first detector field (4a), arranged in a predetermined position at a distance from the light source (1), allows to receive multiply reflected light and provides the said output signal $I_{41}$ from the first detector (4), this signal $I_{41}$ having a substantially linear relationship to the concentration (C) when said concentration is below a critical value of about 1 % and being mainly constant when the concentration (C) is above said critical value,
and in that the output signal $I_{21}$ from the second detector (2) is divided by the output signal $I_{41}$ from the first detector (4), the ratio $I_{21}/I_{41}$ obtained from said dividing representing said measure of the concentration (C) of suspended substances in the flowing medium.

2. A method as claimed in claim 1, **characterized in** that a signal is added to the output

signal $I_{41}$ from said first detector (4) for the multiply reflected light, the former signal being obtained from a part of the light from the light source (1) is led directly and optically to said first detector (4), the ratio being formed according to the relationship $I_{21}/(I_{41} + k \cdot I_1)$, where a detector constant k is obtained by optical means to provide the linearity for the measuring values of the concentration (C) when said concentration is below the critical value of 1% .

3.  A method as claimed in claim 1, **characterized in** that a signal is added to the output signal $I_{41}$ from said first detector (4) for the multiply reflected light, the former signal being obtained from a part of the light from the light source (1) is led directly and electronically to said first detector (4), a measure of the concentration being formed according to the relationship $I_{21}/(I_{41} + \cdot I_1)$, where the constant k is obtained electronically.

4.  A method as claimed in any of the preceding claims, **characterized in** that the light from the field (1a) of the light source (1) is focused at a point outside the window (9) which is between the light field (1a) and the medium flowing in a pipe line (11).

5.  An apparatus for measuring the concentration of suspended substances in a flowing medium, the apparatus comprising:
a probe (10) protruding into a pipe line (11) and being in the form of an enclosure which defines an interior space, the probe (10) having an end portion (13) and a transparent window (9) located in the end portion (13), the transparent window (9) permitting the passage of light therethrough between the interior space and the medium flowing in the pipe line (11) and sealing the interior space from the medium,
a light source (1) connected to a field (1a) in the probe (10) for producing a beam of light for illuminating the medium through the transparent window (9),
a first detector (3;4) connected to a first detector field (3a;4a) in the probe (10), said detector field (3a;4a) being positioned at a distance from the field (1a) associated with the light source (1) so as to intercept multiply reflected light from the medium, the first detector (3;4) including means for producing a first output signal $I_{41}$,
a second detector (2) connected to a second detector field (2a) in the probe (10), the second detector (2) including means for producing a

second output signal $I_{21}$ and
means for combining the first and second output signals $I_{41}$, $I_{21}$ and for producing a measure of concentration (C) of the suspended substances in the flowing medium,
**characterized in** that the second detector field (2a) borders directly on the field (1a) associated with the light source (1) so as to intercept directly reflected light from the medium, the said second output signal $I_{21}$ having a magnitude which varies generally linearly in relation to the concentration (C) of the suspended substances in the flowing medium within the actual measuring range,
the first output signal $I_{41}$ has a magnitude which varies generally linearly in relation to the concentration (C) when said concentration is below a critical value of about 1 % and has a generally constant magnitude when said concentration is above said critical value,
the means for combining the first and second output signals $I_{41}$, $I_{21}$ are electronic and produce said measure of concentration (C) of the suspended substances by dividing the second output signal $I_{21}$ by the first output signal $I_{41}$.

6.  An apparatus as claimed in claim 5, **characterized in** that the field (4a) of the first detector (4) additionally to the measurement of the multiply reflected light is intended to receive a part of the light beam of the light source (1) between the source (1) and the window (9) for measuring the intensity ($I_1$) of the light source (1) before light proceeds into the flowing medium.

7.  An apparatus as claimed in claim 5, **characterized in** that a reflecting means (15) is disposed in the light flow of the light source (1), between the field (1a) associated with the light source (1) and the window (9), said means being adapted for optically conducting a part of the light beam from the field (1a) associated with the light source (1) at right angles to the longitudinal direction of the probe (10) via an obliquely disposed mirror surface (16) into the field (4a) of the first detector (4), said last mentioned measurement taking place additionally to that of the multiply reflected light.

8.  An apparatus as claimed in claim 7, **charactarized in** that the reflecting means consists of the tip (15) of a screw (16) going through the enclosure of the probe (10).

9.  An apparatus as claimed in any of the preceding claims, **characterized in** that the light opening of the field (2a) of the second detector (2) is laterally displaced at the end (13) of the probe (10), at a predetermined distance in relation to the field (2a) of said second detector (2).

10. An apparatus as claimed in any of the preceding claims, **characterized in** that a lens system (22) is disposed in the light beam of the light source (1) between the window (9) and the field (1a) associated with the light source (1), the focal length of said lens system being selected such that the light going through the system (22) is focused at a point beyond the window (9).

## Revendications

1. Un procédé pour mesurer la teneur en substances suspendues d'un milieu en écoulement en illuminant le milieu avec de la lumière a partir d'une source de lumière (1), la lumière étant détectée dans deux détecteurs (2,3 ; 4) par l'intermédiaire de champs de détecteurs (2a,3a;4a), un premier champ de détecteur (4a) étant compris dans un premier (4) desdits détecteurs (2,4) et étant agencé pour mesurer la lumière réfléchie de manière multiple, ladite lumière ayant été réfléchie par les substances en suspension dans le milieu en écoulement, les signaux de sortie $I_{21}$, $I_{41}$ desdits détecteurs (2,3;4) étant influencés par des facteurs de perturbation tels que l'intensité de la source lumineuse et la saleté du verre d'une sonde d'insertion (10), le procédé fournissant le rapport obtenu en divisant les signaux de sortie $I_{21}$,$I_{41}$ provenant desdits deux détecteurs (2,4) en tant que mesure de la concentration (C) des substances en suspension dans le milieu en écoulement, ladite mesure n'étant pas dépendante desdits facteurs de perturbation , caractérisé en ce qu'un deuxième champ de détecteur (2a) compris dans le deuxième (2) desdits détecteurs (2,4) mesure ladite lumière réfléchie directement, ledit second champ de détecteur (2a) étant placé dans une position prédéterminée au voisinage de la source de lumière (1) de sorte que la lumière réfléchie directement soit reçue et qu'une relation sensiblement linéaire soit obtenue dans la plage de mesure effective entre le signal de sortie $I_{21}$ provenant du deuxième détecteur (2) et la concentration (C) des substances en suspension dans le milieu en écoulement, en ce que ledit premier champ de détecteur (4a), placé dans une position prédéterminée a distance de la source de lumière (1), peut recevoir la lumière réfléchie de manière multiple et délivre le signal de sortie $I_{41}$ provenant du premier détecteur (4), ce signal $I_{41}$ présentant une relation sensiblement linéaire par rapport à la concentration (C) lorsque ladite concentration est au-dessous d'une valeur critique d'environ 1% et étant sensiblement constant lorsque la concentration (C) est au-dessus de ladite valeur critique, et en ce que le signal de sortie $I_{21}$ provenant du deuxième détecteur (2) est divisé par le signal de sortie $I_{41}$ provenant du premier détecteur (4), le rapport $I_{21}/I_{41}$ obtenu par ladite division représentant ladite mesure de la concentration (C) des substances en suspension dans le milieu en écoulement.

2. Un procédé selon la revendication 1, caractérisé en ce qu'un signal est ajouté au signal de sortie $I_{41}$ provenant dudit premier détecteur (4) pour la lumière réfléchie de manière multiple, le premier signal étant obtenu à partir d'une partie de la lumière provenant de la source de lumière (1) qui est conduite directement et optiquement audit premier détecteur (4), le rapport étant formé suivant la relation $I_{21}/(I_{41} + k.I_1)$, dans laquelle une constante de détecteur k est obtenue par des moyens optiques pour fournir la linéarité pour les valeurs de mesure de la concentration (C) lorsque ladite concentration est au-dessous de la valeur critique de 1 %.

3. Un procédé selon la revendication 1, caractérisé en ce qu'un signal est ajouté au signal de sortie $I_{41}$ provenant dudit premier détecteur (4) pour la lumière réfléchie de manière multiple, le premier signal étant obtenu à partir d'une partie de la lumière provenant de la source de lumière (1) et étant conduit directement et électroniquement audit premier détecteur (4), une mesure de la concentration étant faite suivant la relation $I_{21}/(I_{41} + k . I_1)$, dans laquelle la constante k est obtenue électroniquement.

4. Un procédé selon l'une quelconque des revendications précédentes, caractérisé en ce que la lumière provenant du champ (1a) de la source de lumière (1) est focalisée en un point à l'extérieur de la fenêtre (9) qui est située entre le champ de lumière (1a) et le milieu s'écoulant dans une conduite (11).

5. Un appareil pour mesurer la teneur en substances suspendues d'un milieu en écoulement, l'appareil comportant : une sonde (10) faisant saillie dans la conduite (11) et se présentant sous la forme d'une enveloppe qui définit un espace intérieur, la sonde (10) présentant une partie extrême (13) et une fenêtre transparente (9) située dans la partie extrême (13), la fenêtre transparente (9) permettant le passage de la lumière à travers elle entre l'espace intérieur et le milieu circulant dans la conduite (11) et fermant de manière étanche l'espace intérieur par rapport au milieu,

une source de lumière (1) reliée à un champ (1a) dans la sonde (10) pour fournir un faisceau de lumière pour illuminer le milieu à travers la fenêtre transparente (9), un premier détecteur (3;4) relié à un premier champ de détecteur (3a;4a) dans la sonde (10), ledit champ de détecteur (3a;4a) étant situé à une distance du champ (1a) associé à la source de lumière (1) de manière à intercepter la lumière réfléchie de manière multiple par le milieu, le premier détecteur (3;4) comportant des moyens pour fournir un premier signal de sortie $I_{41}$,

un second détecteur (2) relié à un second champ de détecteur (2a) dans la sonde (10), le second détecteur (2) comportant des moyens pour fournir un second signal de sortie $I_{21}$, et des moyens pour combiner le premier et le second signaux de sortie $I_{41}$, $I_{21}$ et pour produire une mesure de la concentration (C) des matières en suspension dans le milieu en écoulement,

caractérisé en ce que le second champ de détecteur (2a) borde directement le champ (1a) associé à la source de lumière (1) de manière à intercepter directement 1a lumière réfléchie provenant du milieu, ledit second signal de sortie $I_{21}$ présentant une amplitude qui varie d'une manière générale linéaire avec la concentration (C) des matières suspendues dans le milieu en écoulement, à l'intérieur de la plage effective de mesure,

le premier signal de sortie $I_{41}$ présente une amplitude qui varie d'une manière générale linéaire avec la concentration (C) lorsque ladite concentration est au-dessous d'une valeur critique d'environ 1% et présente une amplitude d'une manière générale constante lorsque ladite concentration est au-dessus de ladite valeur critique,

les moyens pour combiner le premier et le second signaux de sortie $I_{41}$, $I_{21}$ sont électroniques et fournissent ladite mesure de concentration (C) des matières suspendues en divisant le second signal de sortie $I_{21}$ par le premier signal de sortie $I_{41}$.

6.  Un appareil selon la revendication 5, caractérisé en ce que le champ (4a) du premier détecteur (4), en plus de la mesure de la lumière réfléchie de manière multiple, est destiné à recevoir une partie du faisceau de lumière de la source de lumière (1) entre la source (1) et la fenêtre (9) pour la mesure de l'intensité ($I_1$) de la source de lumière (1) avant que la lumière ne pénètre dans le milieu en écoulement.

7.  Un appareil selon la revendication 5, caractérisé en ce que des moyens de réflexion (15) sont disposés dans le trajet de lumière de la source de lumière (1), entre le champ (1a) associé à la source de lumière (1) et la fenêtre (9), lesdits moyens étant agencés pour conduire optiquement une partie du faisceau de lumière provenant du champ (1a) associé à la source de lumière (1) à angle droit par rapport à la direction longitudinale de la sonde (10), par l'intermédiaire d'une surface de miroir (16) disposée obliquement dans le champ (4a) du premier détecteur (4), ladite mesure mentionnée en dernier lieu prenant place additionnellement à celle de la lumière réfléchie de manière multiple.

8.  Un appareil selon la revendication 7, caractérisé en ce que les moyens de réflexion sont constitués par 1'extrémité (15) d'une vis (16) traversant l'enveloppe de la sonde (10).

9.  Un appareil selon l'une quelconque des revendications précédentes, caractérisé en ce que l'ouverture de lumière du champ (2a) du second détecteur (2) est décalée latéralement à l'extrémité (13) de la sonde (10), à une distance prédéterminée par rapport au champ (2a) dudit second détecteur (2).

10. Un appareil selon l'une quelconque des revendications précédentes, caractérisé en ce qu' un système de lentille (22) est disposé dans le faisceau de lumière de la source de lumière (1) entre la fenêtre (9) et le champ (1a) associé à la source de lumière (1), la distance focale dudit système de lentille étant choisie de telle manière que la lumière traversant le système (22) soit focalisée en un point au-delà de la fenêtre (9).

**Ansprüche**

1.  Verfahren zum Messen des Gehaltes an suspendierten Substanzen in einem fließenden Medium durch Beleuchten des Mediums mit Licht (1), welches Licht in zwei Detektoren (2, 3; 4) in Detektionsfeldern (2a, 3a; 4a) detektiert wird, wobei ein erstes Detektionsfeld (4a) in einem ersten (4) der genannten Detektoren (2, 4) inbegriffen ist und zum Messen mehrfach reflektierten Lichtes angeordnet ist, welches Licht von den im fließenden Medium suspendierten Substanzen reflektiert worden ist, und wobei die Ausgangssignale $I_{21}$, $I_{41}$ der genannten Detektoren (2, 3; 4) durch Störfaktoren, wie die Intensität der Lichtquelle und Schmutz am Glas eines Eintauchmeßkopfes (10) beeinflußt werden, bei welchem Verfahren das durch Dividieren der Ausgangssignale $I_{21}$, $I_{41}$ der beiden genannten Detektoren (2, 4) erhaltene Verhältnis als ein Maß der Konzentration (C) von suspendierten Substanzen im fließenden Medium vorgesehen wird, welches Maß nicht von den genannten Störfaktoren abhängig ist, dadurch gekennzeichnet, daß ein zweites Detektionsfeld (2a), welches im zweiten (2) der genannten Detektoren (2, 4) inbegriffen ist, das genannte direkt reflektierte Licht mißt, welches zweite Detektionsfeld (2a) in einer vorbestimmten Lage nahe bei der Lichtquelle (1) angeordnet ist, so daß direkt reflektiertes Licht empfangen wird, und daß ein im wesentlichen linearer Zusammenhang zwischen dem Ausgangssignal $I_{21}$ des zweiten Detektors (2) und der Konzentration (C) der suspendierten Substanzen im fließenden Medium innerhalb des Ist-Wert Meßbereiches erhalten wird, daß das genannte erste Detektionsfeld (4a), das in einer vorbestimmten Lage in einem Abstand von der Lichtquelle (1) angeordnet ist, mehrfach reflektiertes Licht aufnehmen kann, und das genannte Ausgangssignal $I_{41}$ des ersten Detektors (4) bildet, wobei dieses Signal $I_{41}$ einen im wesentlichen linearen Zusammenhang mit der Konzentration (C) hat, wenn die genannte Konzentration unter einem kritischen Wert von ungefähr 1% liegt, und im wesentlichen konstant ist, wenn die Konzentration (C) über diesem kritischen Wert liegt, und daß das Ausgangssignal $I_{21}$ des zweiten Detektors (2) durch das Ausgangssignal $I_{41}$ des ersten Detektors (4) dividiert wird, wobei das aus diesem Dividieren erhaltene Verhältnis $I_{21}/I_{41}$ das genannte Maß für die Konzentration (C) von suspendierten Substanzen im fließenden Medium darstellt.

2. Verfahren nach Anspruch 1, dadurch gekennzeichnet, daß ein Signal zum Ausgangssignal $I_{41}$ des genannten ersten Detektors (4) für das mehrfach reflektierte Licht hinzugefügt wird, wobei das ersterwähnte von einem Teil des Lichtes der Lichtquelle (1) erhaltene Signal direkt und optisch zum genannten ersten Detektor (4) geführt wird, und wobei das Verhältnis gemäß der Beziehung $I_{21}/(I_{41} + k \cdot I_1)$ gebildet wird, wobei eine Detektorkonstante k auf optischem Weg erhalten wird, um die Linearität für die Meßwerte der Konzentration (C) zu schaffen, wenn die genannte Konzentration unter dem kritischen Wert von 1% liegt.

3. Verfahren nach Anspruch 1, dadurch gekennzeichnet, daß ein Signal zum Ausgangssignal $I_{41}$ des genannten ersten Detektors (4) für das mehrfach reflektierte Licht hinzugefügt wird, wobei das ersterwähnte von einem Teil des Lichtes der Lichtquelle (1) erhaltene Signal, direkt und elektronisch zum genannten ersten Detektor (4) geführt wird, und eine Maß der Konzentration gemäß der Beziehung $I_{21}/(I_{41} + k \cdot I_1)$ gebildet wird, wobei die Konstante k elektronisch erhalten wird.

4. Verfahren nach einem der vorhergehenden Ansprüche, dadurch gekennzeichnet, daß das Licht vom Feld (1a) der Lichtquelle (1) auf einen außerhalb des Fensters (9), welches sich zwischen dem Lichtfeld (1a) und dem in einer Rohrleitung (11) fließenden Medium befindet, liegenden Punkt fokussiert wird.

5. Vorrichtung zum Messen der Konzentration von suspendierten Substanzen in einem fließenden Medium, welche Vorrichtung:
einen Meßkopf (10), der in eine Rohrleitung (11) ragt und die Form eines Gehäuses, das einen Innenraum umgrenzt, hat, welcher Meßkopf (10) einen Endteil (13) und ein im Endteil (13) liegendes transparentes Fenster (9) hat, welches transparente Fenster (9) den Durchgang von Licht zwischen dem Innenraum und dem in der Rohrleitung (11) fließenden Medium gestattet und den Innenraum gegenüber dem Medium abdichtet,
eine Lichtquelle (1), welche mit einem Feld (1a) im Meßkopf (10) zur Bildung eines Lichtstrahls zum Beleuchten des Mediums durch das transparente Fenster (9) verbunden ist,
einen ersten Detektor (3; 4), der mit einem ersten Detektionsfeld (3a; 4a) im Meßkopf (10) verbunden ist, welches Detektionsfeld (3a; 4a;) in einem Abstand von dem der Lichtquelle (1) zugeordneten Feld (1a) positioniert ist, um mehrfach reflektiertes Licht vom Medium aufzufangen, wobei der erste Detektor (3; 4) Mittel zur Bildung eines ersten Ausgangssignals $I_{41}$ aufweist,
und einen zweiten Detektor (2), der mit einem zweiten Detektionsfeld (2a) im Meßkopf (10) verbunden ist, wobei der zweite Detektor (2) Mittel zur Bildung eines zweiten Ausgangssignals $I_{21}$ und Mittel zum Kombinieren der ersten und zweiten Ausgangssignale $I_{41}$, $I_{21}$ und zur Bildung eines Maßes der Konzentration (C) der suspendierten Substanzen im fließenden Medium beinhaltet,

aufweist, welche Vorrichtung dadurch gekennzeichnet ist, daß das zweite Detektionsfeld (2a) direkt an das mit der Lichtquelle (1) in Verbindung stehende Feld (1a) grenzt, um vom Medium direkt reflektiertes Licht aufzufangen, wobei das genannte zweite Ausgangssignal $I_{21}$ eine Größe hat, welche sich im allgemeinen linear in Beziehung zur Konzentration (C) der suspendierten Substanzen im fließenden Medium im Ist-Wert Meßbereich ändert, wobei das erste Ausgangssignal $I_{41}$ eine Größe hat, welche sich im allgemeinen linear in Beziehung zur Konzentration (C) ändert, wenn die genannte Konzentration unter einem kritischen Wert von ungefähr 1% liegt, und eine im allgemeinen konstante Größe hat, wenn die genannte Konzentration über dem genannten kritischen Wert liegt,

und die Mittel zum Kombinieren der ersten und zweiten Ausgangssignale $I_{41}$, $I_{21}$ elektronische Mittel sind und das Maß der Konzentration (C) der suspendierten Substanzen durch Dividieren des zweiten Ausgangssignales $I_{21}$ durch das erste Ausgangssignal $I_{41}$ bilden.

6. Vorrichtung nach Anspruch 5, dadurch gekennzeichnet, daß das Feld (4a) des ersten Detektors (4) zusätzlich zur Messung des mehrfach reflektierten Lichtes zum Empfangen eines Teiles des Lichtstrahles der Lichtquelle (1) zwischen der Quelle (1) und dem Fenster (9) zum Messen der Intensität ($I_1$) der Lichtquelle (1) bevor das Licht in das fließende Medium gelangt, vorgesehen ist.

7. Vorrichtung nach Anspruch 5, dadurch gekennzeichnet, daß ein Reflexionsmittel (15) im Lichtstrom der Lichtquelle (1) zwischen dem der Lichtquelle zugeordneten Feld (1a) und dem Fenster (9) angeordnet ist, welches Mittel dazu eingerichtet ist, einen Teil des Lichtstrahles von dem der Lichtquelle (1) zugeordneten Feld (1a) optisch im rechten Winkel zur Längsrichtung des Meßkopfes (10) durch eine schräg angeordnete Spiegelfläche (16) in das Feld (4a) des ersten Detektors (4) zu führen, wobei die letzterwähnte Messung zusätzlich zu jener des mehrfach reflektierten Lichtes stattfindet.

8. Vorrichtung nach Anspruch 7, dadurch gekennzeichnet, daß das Reflexionsmittel aus der Spitze (15) einer Schraube (16) besteht, welche durch das Gehäuse des Meßkopfes (10) hindurchgeht.

9. Vorrichtung nach einem der vorhergehenden Ansprüche, dadurch gekennzeichnet, daß die Lichtöffnung des Feldes (2a) des zweiten Detektors (2) in einem vorbestimmten Abstand im Verhältnis zum Feld (2a) des genannten zweiten Detektors (2) am Ende (13) des Meßkopfs (10) seitlich versetzt ist.

10. Verfahren nach einem der vorhergehenden Ansprüche, dadurch gekennzeichnet, daß ein Linsensystem (22) im Lichtstrahl der Lichtquelle (1) zwischen dem Fenster (9) und dem der Lichtquelle (1) zugeordneten Feld (1a) angeordnet ist, wobei die Brennweite des genannten Linsensystems so gewählt ist, daß das durch das System (22) gehende Licht an einem jenseits des Fensters (9) liegenden Punkt fokussiert ist.

Fig.1

Fig.2

Fig.1A

Fig.3

Fig.4

Fig.5

Fig.6

# Fig. 7

# Fig. 8